# EUROPEAN PATENT SPECIFICATION

(11) **EP 0 910 339 B1**
(45) Date of publication and mention of the grant of the patent: **02.02.2005**
(21) Application number: 97914779.0
(22) Date of filing: 21.02.1997
(51) Int. Cl.: A61K 9/00

(54) **BUCCAL POLAR SPRAY**
POLARES BUKKALES SPRAY
AEROSOL BUCCAL POLAIRE

(30) Priority: 12.04.1996 US 630065
(43) Date of publication of application: 28.04.1999
(73) Proprietor: Novadel Pharma Inc., Flemington, NJ 08822 (US)
(72) Inventor: DUGGER, Harry, A., III, Flemington, NJ 08822 (US)
(74) Representative: Luderschmidt, Schüler & Partner GbR
(86) International application number: PCT/US1997/002793
(87) International publication number: WO 1997/038662

(56) References cited:
- EP-A- 0 315 960
- EP-A- 0 557 129
- EP-A- 0 719 549
- WO-A-94/13280
- DE-A- 3 338 978
- DE-A- 3 544 692
- DE-A- 3 922 650
- US-A- 3 784 684
- US-A- 4 232 002
- US-A- 4 935 243
- US-A- 5 135 753
- US-A- 5 456 677
- US-A- 5 593 684

## Description

It is known that certain biologically active compounds are better absorbed through the oral mucosa than through other routes of administration, such as through the stomach or intestine. However, formulations suitable for such administration by these latte routes present their own problems. For example, the biologically active compound must be compatible with the other components of the composition such as propellants, solvents, etc. Many such formulations have been proposed. For example, US-A-4,689,233, Dvorsky et al., describes a soft gelatine capsule for the administration of the anti-coronary drug nifedipine dissolved in a mixture of polyether alcohols. US-A-4,755,389, Jones et al., describes a hard gelatin chewable capsule containing nifedipine. A chewable gelatin capsule containing a solution or dispersion of a drug is described in US-A-4,935,243, Borkan et al. US-A-4,919,919, Aouda et al. and US-A-5,370,862, Klokkers-Bethke, describe a nitroglycerin spray for administration to the oral mucosa comprising nitroglycerin, ethanol, and other components. An orally administered pump spray is described by Cholcha in US-A-5,186,925. Aerosol compositions containing a hydrocarbon propellant and a drug for administration to a mucosal surface are described in GB-A-2,082,457, Su, US-A-3,155,574, Silson et al., US-A-5,011,678, Wang et al., and by Parnell in US-A-5,128,132. It should be noted that these references discuss bioavailability of solutions by inhalation rather than through the membranes to which they are administered.

DE-A-39 22 650 discloses a spray composition containing nitroglycerin as a polar solvent which is known to be well absorbed through many membranes including plastics and the oral mucosa. Nitroglycerin has a low molecular weight and is highly fat soluble. Nitroglycerin is known as one of the drugs most rapidly transported across the skin barrier even without a penetration enhancer. DE-A-35 44 692 describes liquid compositions for administration for patients with blood circulation disturbance. It is well tolerated by throat mucous membrane. DE-A-33 38 978 describes a composition for a mouth spray containing verapimil or gallopamil and as a solvent a mixture of about 7.3 weight % ethanol and about 90 weight % water. WO-A-94/13280 discloses a sprayable analgesic composition for absorption through the buccal mucosa into the blood stream. EP-A-0 557 129 discloses nicotine in liquid form dispersed in at least one pharmaceutically acceptable carrier. The formulation contains nicotine, alcohol, saccharine, glycerine and peppermint flavour. US-A-5,456,677 discloses a method and apparatus for easily administering caffeine in combination with a breath freshener.

A buccal aerosol spray using a polar solvent has now been developed which provides biologically active compounds for rapid absorption through the oral mucosa, resulting in fast onset of effect.

The buccal aerosol spray compositions of the present invention, for transmucosal administration of a pharmacologically active compound soluble in a pharmacologically acceptable polar solvent comprising in weight% of total composition: polar solvent 75-99.8%, active compound 0.0025-40%, suitably additionally comprising, by weight of total composition a flavoring agent 0.05-5%. Preferably the composition comprises: polar solvent 75-99%, active compound 0.025-20%, flavoring agent 0.1-2.5%; most suitably polar solvent 75-98%, active compound 0.125-12.5%, flavoring agent 0.1-2.5%.

It is an object of the invention to coat the mucosal membranes with extremely fine droplets of spray containing the active compounds

A further object is a pump spray container containing a composition of the spray formulation, and a metered valve suitable for releasing from said container a predetermined amount of said composition.

The solvent is a low molecular weight, pharmacologically acceptable alcohol, water, glycerine or polyethylene glycol or mixtures thereof.

(All percentages herein are by weight unless otherwise indicated.)

The spray compositions of the invention are intended to be administered from a pump spray.

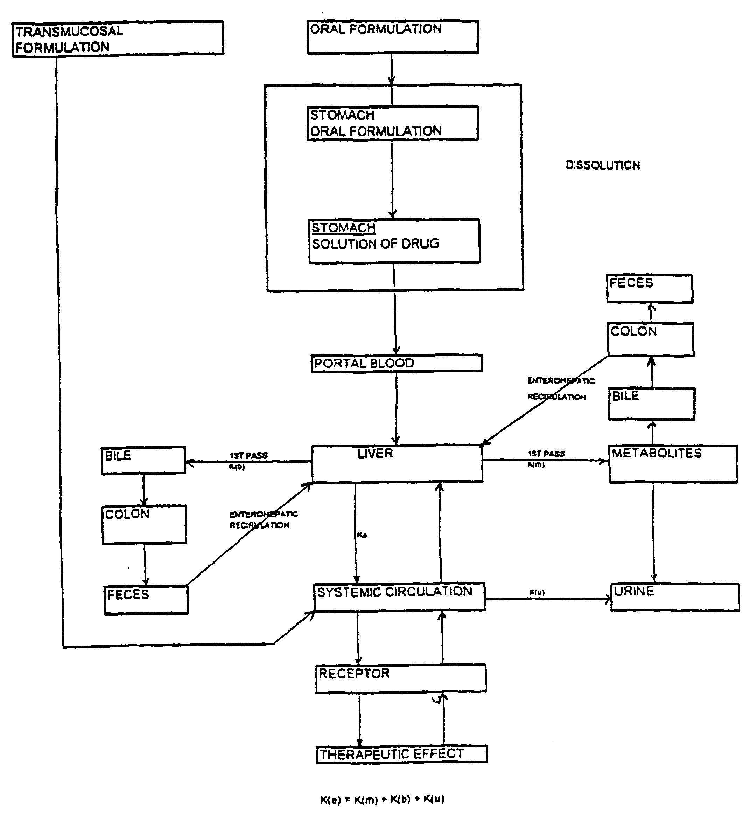

### BRIEF DESCRIPTION OF THE DRAWING

The figure is a schematic diagram showing routes of absorption and processing of pharmacologically active substances in a mammalian system.

### DESCRIPTION OF THE PREFERRED EMBODIMENTS

The preferred active compounds of the present invention are nicotine, clemastine, testosterone, estradiol, progesterone, fluoxetine, and piroxicam in their nonionized form or as the free base of the pharmaceutical acceptable salts thereof (provided, for the aerosol or spray compositions, they are soluble in the spray solvent). These compounds are soluble in the polar solvents of the invention at useful concentrations. These concentrations may be less than the standard accepted dose for these compounds since there is enhanced absorption of the compounds through the oral mucosa. This aspect of the invention is especially important when there is a large (40-99.99%) first pass effect.

As solvents for the sprays there may be used low molecular weight polyethyleneglycols (PEG) of 400-1000 Mw (preferably 400-600). Low molecular weight alcohols and polyols, such as glycerin may also be present and water may also be used.

The preferred flavoring agents are synthetic or natural oil of peppermint, oil of spearmint, citrus oil, fruit flavors, chocolate, sweeteners (sugars, aspartame, saccharin, etc.), and combinations thereof.

The active substances include the active compounds selected from the group consisting of alkaloids, anti histamines, steroid hormones, and anti-depressants, benzodiazepines, such as tamezepam.

Clemastine hydrogen fumarate is a known (Tavist®, Sandoz) anti-histamine. The spray of the invention advantageously coats the oral mucosa with an immediately available dose of clemastine which can be rapidly absorbed. This is highly desirable, as during an acute asthma attack.

Nicotine is a component of tobacco products which is considered addictive. Smokers wishing to stop smoking have a dual problem. First, is the addictive properties of nicotine itself. Second, is that the habit is associated with smoking activities, i.e., puffing, inhaling, etc. The spray of the invention dissociates these two problems. By presenting nicotine in a form which can be readily absorbed, the spray allows the smoker to temporarily continue nicotine use but terminate smoking. Once the habit of smoking is stopped, the former smoker can then be weaned off nicotine use, as by less frequent use and/or by use of a lower concentration spray. Advantageously, during this regimen, the user is exposed to none of the carcinogens present in tobacco smoke.

Testosterone is a hormone produced by gonadal cells. Testosterone, especially the esters thereof (e.g., acetate, propionate, enanthate, and cypionate), is used in the treatment of hypogonadism.

Estradiol is an estrogen steroid secreted from the ovaries. Estradiol, especially the esters thereof (e.g., diacetate, and benzoate), is used as estrogen replacement therapy, especially in post-menopausal women.

Progesterone is a hormone produced by the corpus luteum. Fluoxetine is an antidepressant also known as Prozac. Piroxicam is a known (Feldene®, Pfizer) anti-inflammatory.

The formulations of the present invention comprise an active compound or a pharmaceutically acceptable salt thereof. The term "pharmaceutically acceptable salts" refers to salts prepared from pharmaceutically acceptable non-toxic acids or bases including organic and inorganic acids or bases.

When an active compound of the present invention is acidic, salts may be prepared from pharmaceutically acceptable non-toxic bases. Salts derived from all stable forms of inorganic bases include aluminum, ammonium, calcium, copper, iron, lithium, magnesium, manganese, potassium, sodium, zinc, etc. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines and basic ionexchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, methylglucosamine, morpholine, piperazine, piperidine, polyamine resins, procaine, purine, theobromine, triethylamine, trimethylamine, tripropylamine, etc.

When an active compound of the present invention is basic, salts may be prepared from pharmaceutically acceptable non-toxic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, mandelic, methanesulfonic, mucic, nitric, pamoic, pantothenic, phosphoric, succinic, sulfuric, tartaric, p-toluenesulfonic, etc. Particularly preferred are citric, hydrobromic, maleic, phosphoric, sulfuric, and tartaric acids.

In the discussion of the manufacture of buccal aerosol pump sprays herein, reference to the active compounds is meant to also include the pharmaceutically acceptable salts thereof. While certain formulations are set forth herein, the actual amounts to be administered to the mammal or man in need of same are to be determined by the treating physician.

The invention is further defined by reference to the following examples, which are intended to be Illustrative and not limiting.

### EXAMPLE 1

### Clemastine Spray

A spray of the invention comprises the following formulation:

| | Amount | Preferred Amount | Most-Preferred Amount |
|---|---|---|---|
| Polar solvent | 75-99% | 90-99% | 97-99% |
| Clemastine fumarate | 0.12-12.5% | 0.25-7.25% | 0.25-6.5% |
| Flavoring agent | 0.05-5% | 0.1-2.5% | 0.2-2% |

It is particularly preferred to formulate the spray delivering 1.34mg/activation:

| | Amount |
|---|---|
| Polar solvent: | |
| Ethanol | 66% |
| Water | 31% |
| Clemastine fumarate | 2.8% |
| Peppermint Oil | 0.2% |

### EXAMPLE 2

### Nicotine Spray

A spray of the invention comprises the following formulation:

| | Amount | Preferred Amount | Most-Preferred Amount |
|---|---|---|---|
| Polar solvent | 75-99.8% | 96-99.8% | 96-99.8% |
| Nicotine | 0.0125-10% | 0.125-2.5% | 0.2-1.15% |
| Flavoring agent | 0.05-5% | 0.1-2.5% | 0.2-2.0% |

It is particularly preferred to formulate the spray delivering 0.5mg/activation:

| | Amount |
|---|---|
| Polar solvent: | |
| Ethanol | 49.37% |
| Water | 49.37% |
| Nicotine | 1.06% |
| Peppermint Oil | 0.2% |

### EXAMPLE 3

### Nicotine Sulfate

A spray of the invention comprises the following formulation:

| | Amount | Preferred Amount | Most-Preferred Amount |
|---|---|---|---|
| Polar solvent | 75-99.8% | 96-99.8% | 96-99.8% |
| Nicotine Sulfate | 0.0125-10% | 0.125-2.5% | 0.2-2.0% |
| Flavoring agent | 0.05-5% | 0.1-2.5% | 1-2% |

It is particularly preferred to formulate the spray for 0.5mg of nicotine sulfate:

| | Amount |
|---|---|
| Polar solvent: | |
| Ethanol | 9.84% |
| Water | 88.9% |
| Nicotine Sulfate | 1.06% |
| Peppermint Oil | 0.2% |

### EXAMPLE 4

### Fluoxetine Hydrochloride Spray

A spray of the invention comprises the following formulation:

| | Amount | Preferred Amount | Most-Preferred Amount |
|---|---|---|---|
| Polar solvent | 75-99% | 75-98% | 75-95% |
| Fluoxetine Hydrochloride | 0.125-25% | 2.5-20% | 5-12.5% |
| Flavoring agent | 0.05-5% | 0.1-2.5% | 0.1-2.0% |

It is particularly preferred to formulate the spray delivering 5mg/activation:

| | Amount |
|---|---|
| Polar solvent: | |
| Ethanol | 48.4% |
| Water | 10.0% |
| Polyethyleneglycol | 30.0% |
| Fluoxetine HCl | 10.6% |
| Oil of Orange | 1.0% |

### EXAMPLE 5

### Testosterone Spray Delivering 3mg/Activation

A spray of the invention comprises the following formulation:

| | Amount | Preferred Amount | Most-Preferred Amount |
|---|---|---|---|
| Polar Solvent | 55-99% | 75-95% | 85-93% |
| Testosterone | 0.12-10% | 0.25-7.5% | 0.25-6.5% |
| Flavoring Agent | 0.05-3% | 0.1-2.5% | 0.1-2.5% |

It is particularly preferred to formulate the spray:

| | Amount |
|---|---|
| Polar Solvent: | |
| Water | 10% |
| Polyethyleneglycol | 65% |
| Ethanol | 16.6% |
| Testosterone | 6.4% |
| Orange Aroma | 1.0% |
| Oil of Citrus | 1.0% |

### EXAMPLE 6

### Estradiol Spray Delivering 0.1 mg/Activation

A spray of the invention comprises the following formulation:

| | Amount | Preferred Amount | Most-Preferred Amount |
|---|---|---|---|
| Polar Solvent | 75-99% | 75-95% | 85-99% |
| Estradiol | 0.0025-2.5% | 0.025-1.5% | 0.125-1.0% |
| Flavoring Agent | 0.05-3% | 0.1-2.5% | 1-2% |

It is particularly preferred to formulate the spray:

| | Amount |
|---|---|
| Polar Solvent: | |
| Water | 10% |
| Polyethyleneglycol | 75% |
| Ethanol | 13.79% |
| Estradiol | 0.21% |
| Peppermint | 1.0% |

### EXAMPLE 7

### Progesterone Spray Delivering 0.32mg/Activation

A spray of the invention comprises the following formulation:

| | Amount | Preferred Amount | Most-Preferred Amount |
|---|---|---|---|
| Propellent | 55-99% | 75-99.8% | 85-99.5% |
| Progesterone | 0.12-10% | 0.25-6.25% | 0.25-1% |
| Flavoring Agent | 0.05-3% | 0.1-2.5% | 1-2% |

It is particularly preferred to formulate the spray:

| | Amount |
|---|---|
| Polar Solvent: | |
| Water | 10% |
| Polyethyleneglycol | 75% |
| Ethanol | 13.32% |
| Progesterone | 0.68% |
| Peppermint | 1.0% |

## Claims

1. Use of a pharmacologically active compound selected from the group consisting of non-steroidal anti-inflammatories, anti-histamines, steroid hormones, benzodiazepines and anti-depressants for the preparation of a buccal aerosol pump spray composition for being absorbed through the oral mucosa, said spray comprising in weight % of total composition: polar solvent 75 - 99,8 % and active compound 0,0025 - 20 %.

2. Use of the pharmacologically active compound of claim 1, wherein the solvent is selected from the group consisting of alcohols of C₇₋₁₈ hydrocarbons of a linear or branched configuration.

3. Use of the pharmacologically active compound of claim 1, wherein the active compound is selected from the group consisting of clemastine, testosterone, estradiol, progesterone, temazepam and fluoxetine in their non-ionized form or as the free base of the pharmaceutically acceptable salts thereof.

4. Use of the pharmacologically active compound of claim 1, wherein the spray additionally comprises flavouring agents selected from the group consisting of synthetic or natural oil of peppermint, oil of spearmint, citrus oil, fruit flavors, sweeteners and combinations thereof.

## Patentansprüche

1. Verwendung einer pharmazeutisch-aktiven Verbindung aus der Gruppe, bestehend aus nicht-steroidalen Antirheumatika, Antihistaminen, Steroidhormonen, Benzodiazepinen und Antidepressiva, für die Herstellung einer bukkalen Aerosolpumpspray-Zusammensetzung, die durch die Mundschleimhaut absorbiert werden soll, wobei das Spray, in Gew.-% bezogen auf die Gesamtzusammensetzung, 75 - 99,8 % polares Lösungsmittel und 0,0025 -20 % aktive Verbindung umfasst.

2. Verwendung der pharmazeutisch-aktiven Verbindung nach Anspruch 1, wobei das Lösungsmittel ein Alkohol eines C₇₋₁₈ Kohlenwasserstoffes mit linearer oder verzweigter Konfiguration ist.

3. Verwendung der pharmazeutisch-aktiven Verbindung nach Anspruch 1, wobei die aktive Verbindung Clemastin, Testosteron, Estradiol, Progesteron, Temazepam oder Fluoxetin, in nicht-ionisierter Form oder als freie Base eines pharmazeutischannehmbaren Salzes davon, ist.

4. Verwendung der pharmazeutisch-aktiven Verbindung nach Anspruch 1, wobei das Spray weiterhin synthetisches oder natürliches Pfefferminzöl, Spermintöl, Zitronenöl, Fruchtaroma, Süssstoffe oder Kombinationen davon als Aromastoffe umfasst.

## Revendications

1. Utilisation d'un composé pharmacologiquement actif choisi parmi le groupe constitué par des anti-inflammatoires non stéroïdiens, des antihistaminiques, des hormones stéroïdiennes, des benzodiazépines et des antidépresseurs pour la préparation d'une composition buccale de bouffée d'aérosol à pompe destinée à être absorbée via la muqueuse buccale, ladite bouffée comprenant, en % en poids de la composition totale, un solvant polaire à concurrence de 75 à 99,8 % et un composé actif à concurrence de 0,0025 à 20 %.

2. Utilisation du composé pharmacologiquement actif selon la revendication 1, dans laquelle le solvant est choisi parmi le groupe constitué par des alcools d'hydrocarbures en C₇-C₁₈ de configuration linéaire ou ramifiée.

3. Utilisation du composé pharmacologiquement actif selon la revendication 1, dans laquelle le composé actif est choisi parmi le groupe constitué par la clémastine, la testostérone, l'oestradiol, la progestérone, le témazépam et la fluoxétine sous leur forme non ionisée ou sous forme de la base libre de leurs sels pharmaceutiquement acceptables.

4. Utilisation du composé pharmacologiquement actif selon la revendication 1, dans laquelle la bouffée comprend en outre des agents aromatisants choisis parmi le groupe constitué par de l'essence de menthe poivrée synthétique ou naturelle, de l'essence de menthe verte, de l'huile d'agrumes, des arômes de fruits, des édulcorants et leurs combinaisons.
